# EUROPEAN PATENT APPLICATION

(11) **EP 0 539 315 A2**
(43) Date of publication of application: **28.04.1993**
(21) Application number: 92500134.9
(22) Date of filing: 20.10.1992
(51) Int. Cl.: A61C 19/00, A61C 1/16, A61B 19/08

(54) **Disposable sheath for a dental optical-fiber lighting-system**

(30) Priority: 21.10.1991 ES 9103132
(71) Applicant: Sastre Montalvo, Alberto, Cartagena (Murcia) (ES)
(72) Inventor: Sastre Montalvo, Alberto, Cartagena (Murcia) (ES)
(74) Representative: Garcia Cabrerizo, Francisco

(57) **Abstract**

A disposable flexible sheath (1), slidable on the halogen light optical fiber tube (2), having a transparent closed distal end (2'), to be used during dental treatments.

The sheath is cylinder shaped, its peripheral walls (11) being made of flexible, thin materials such as plastic, plasticised paper, rubber or similar.

## Description

### PURPOSE OF THE INVENTION

This descriptive report refers to a disposable cover for a halogen light optical fibre tube to be used in dentistry whose evident purpose is to constitute an hygienic element which is fitted onto the optical fibre tube, introduced in the oral cavity and which is applied onto the composite filling, directly inducing its hardening, avoiding as a consequence of the cover itself, the pressing need to sterilise conventional tubes existing at present.

### FIELD OF APPLICATION OF THE INVENTION

This invention can be applied within the industry engaged in the manufacture of elements and accessories for estomatology.

### DESCRIPTION OF THE INVENTION

This disposable cover for halogen light optical fibre tubes to be used in dentistry suggested by this invention constitutes by itself an obvious solution to the problem existing at present with regard to this matter.

More specifically, the disposable cover for halogen light optical fibre tubes to be used in dentistry, the object of this invention, is constituted by the manufacture of a cover, made with a plastic material, which adopts a shape similar to that of the finger of a disposable glove which can be fitted to the surface of the tube thus sterilising it completely and rendering it useful to make it possible for the patient to undergo the filling of a teeth, this cover being taken away once the tube has been used and the corresponding operations in the patient's oral cavity are finished.

When the time comes to perform a new operation on a different patient, the specialist will apply on the surface of the tube a new cover, thus guaranteeing the hygiene or asepsis of the same, endowing the conventional item with a set of possibilities completely unknown, and consequently avoiding the traditional sterilisation.

This cover must fulfil the condition of being transparent, specially through the area through which the light flowing from the optical fibre tube comes. The ideal material to be used should be a kind of transparent plasticized paper like the one used to make aesthetic fillings and which is placed in front of the beam of the halogen light, staying between the filling and the tube.

A fine plastic completely transparent can also be used.

The disposable cover is universal for all the types of tubes in the market, because the difference existing between these is very slight, and those with the thickest dimensions can be used as a pattern, because even though for the models with a smaller section they could fit looser this doesn't involve any inconvenience because they allow the performance of a completely adequate operation.

Equally, there is also the possibility to choose as a cover an aseptic rubber tube or a similar plastic material with a higher thickness, as far as the material itself is concerned, made with a certain degree of flexibility, and of course with a completely transparent cover on its blind end.

### DESCRIPTION OF THE DRAWINGS

To complement the description in progress and with the aim to aid to a better understanding of the features of the invention, we enclose to this descriptive report, constituting part of it, a sheet of plans on which are shown, with a descriptive and non limitative character the following items:

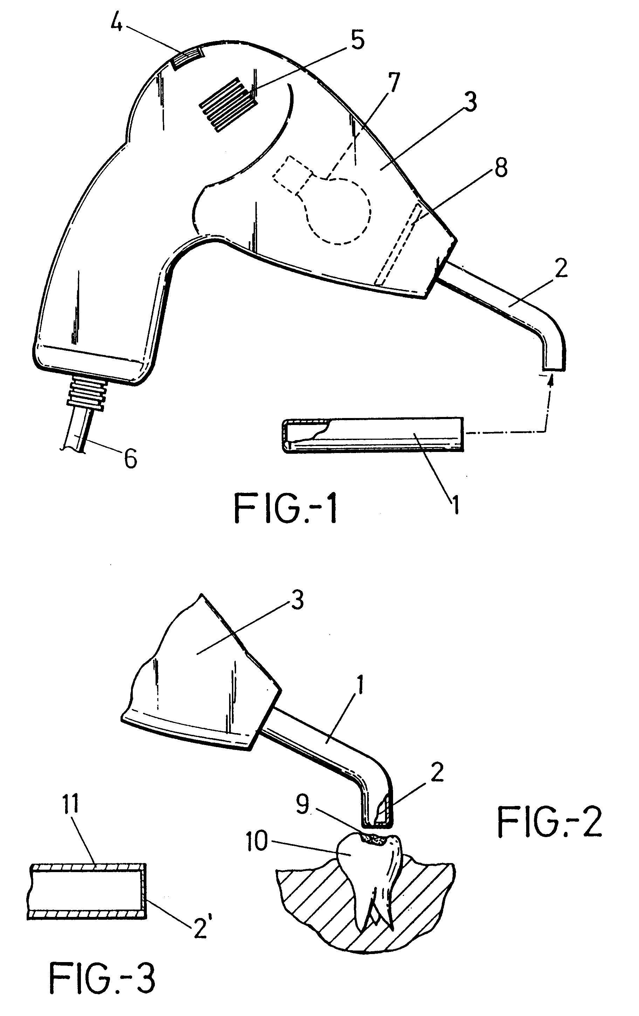

Figure 1.- Shows a side elevation of the disposable cover for optical fiber tube to be used in estomatology, the subject of the invention, together with particulars of the above cover.

Figure 2.- shows a view of the optical fiber tube which fits the cover object of the invention on the specific area.

Figure 3.- Shows the details of a cover with identical features to the proposed, but made with a thicker material.

### DESCRIPTION OF THE INVENTION

From these drawings it can be observed in which way is shaped the disposable cover for halogen light optical fiber tube to be used in estomatology, from the utilisation of a useful light generator (3), furnished with ventilation grids (4) and (5), a halogen light bulb in the inside (7) and with a blue filter (8), receiving the power supply by means of a cable (6).

The cover (1) is fitted to the external surface of an optical fiber tube (2) through which the blue halogen light flowing from the lamp (7) comes out, masking the whole tube the piece that constitutes the cover (1) itself.

When the time comes to use this device to apply the pertinent filling (9) into a tooth (10), the specialist, letting aside the sterilization will fit the cover (1) onto the tube (2) and will perform the corresponding work.

Once finished, the specialist will take the cover away (1) and the tool will be ready to receive a new cover should it be used in another oral cavity.

This invention takes into.account the possibility that the cover (1) has a higher thickness shaping it as a tube made with flexible rubber (11) with a transparent area on the blind end (2) through which the halogen light will come out.

We do not deem it necessary to extend ourselves in the description because any expert in the matter will understand the scope of the invention and the advantages derived from it.

The materials, shape, size and arrangement of the elements will be subject to changes provided that they do not alter the essence of the invention.

"The words used to describe this report will be always taken in the broadest and non restrictive sense of the term".

## Claims

**1st.** Disposable cover for halogen light optical fiber tubes to be used in estomatology, from the type utilised on the external surface of an optical fiber tube through which the light of a halogen bulb fitted on a light emitting device flows out. This is used to put into practice the filling technique on a tooth and its main feature is that it uses a flexible material such as plasticized paper, thin plastic, flexible rubber or the like, having the shape of a cylindrical element with a completely open end whereas the other end is blind and has a completely transparent surface.
